# EUROPEAN PATENT APPLICATION

(11) **EP 4 806 425 A1**
(43) Date of publication of application: **16.09.2026**
(21) Application number: 25163674.2
(22) Date of filing: 13.03.2025
(51) Int. Cl.: A61K 9/00, A61K 31/19, A61K 31/485, A61P 25/00, A61P 43/00

(54) **COMPOSITIONS COMPRISING VALPROIC ACID FOR USE IN IMPROVING MEMORY**

(71) Applicant: Lu, Ru-Band, New Taipei City 241 (TW)
(72) Inventor: Lu, Ru-Band, New Taipei City 241 (TW)
(74) Representative: Lang, Christian

(57) **Abstract**

The present invention provides a composition for use in a method of improving cognitive function of a subject, wherein the active ingredient of the composition comprises valproic acid, dextromethorphan or a combination thereof.

## Description

### FIELD OF THE INVENTION

The present invention provides a composition for use in improving cognitive function of a subject, which is characterized by using dextromethorphan or valproic acid, or a combination of dextromethorphan and valproic acid for treatment.

### DESCRIPTION OF PRIOR ART

Bipolar disorder is a severe and disabling psychiatric disorder that encompasses a large group of clinical manifestations, and the main symptoms comprise mood disorders, specific risk-taking behaviors, impulsivity, interpersonal disorders, and depression. According to Diagnostic and Statistical Manual of Mental Disorders, Fifth Edition (DSM-5), bipolar disorder can be divided into four types: bipolar I disorder, bipolar II disorder, cyclothymic disorder, and bipolar disorder not otherwise specified.

The symptoms of bipolar II disorder are a combination of depressive episodes and hypomanic episodes. Bipolar II disorder is often misdiagnosed because the patients frequently express depressive episodes but the period of the hypomanic episodes is short and the symptoms thereof are less obvious. Therefore, the patients often do not mention these symptoms during their doctor visits, and the doctor will neglect to inquire about their medical history to ignore the condition. As a result, patients with bipolar II disorder often do not receive a correct diagnosis and effective treatment. Compared with patients with bipolar I disorder, patients with bipolar II disorder have the same or higher risk of suicide, more frequent episodes of emotional syndromes, and more episodes of depression.

Many studies have shown that patients with bipolar disorder have a wide range of neuropsychological impairments during the symptom period, including executive function, sustained attention, working memory, verbal memory, verbal fluency, cognitive resilience, abstract reasoning and visuomotor skills, visuospatial abilities, and general cognitive function.

Therefore, it is a major issue in clinical treatment how to improve the neuropsychological impairments of patients with bipolar disorder.

### DETAILED DESCRIPTION OF THE INVENTION

As used herein, the term "a" or "an" are employed to describe elements and components of the present invention. This is done merely for convenience and to give a general sense of the present invention. This description should be read to include one or at least one and the singular also includes the plural unless it is obvious that it is meant otherwise.

The term "or" as used herein may mean "and/or."

As used herein, "cognitive function" means an intellectual process by which one becomes aware of, perceives, or comprehends ideas. Cognitive function involves all aspects of perception, thinking, reasoning, memory, and attention. In one embodiment, the cognitive function comprises global cognitive function, sustained cognition, memory, language, executive function, and attention. In a preferred embodiment, the attention comprises sustained attention.

The present invention provides a use of a composition for preparing a drug for improving sustained attention of a subject, wherein the active ingredient of the composition comprises dextromethorphan.

In addition, the present invention also provides a use of a composition for preparing a drug for improving memory of subject, wherein the active ingredient of the composition comprises valproic acid or a pharmaceutically acceptable salt thereof.

The present invention further provides a composition for use in a method of improving memory of a subject, wherein the active ingredient of the composition comprises valproic acid or a pharmaceutically acceptable salt thereof.

As used herein, the term "pharmaceutically acceptable salt" refers to salts that are suitable for tissue contact with humans and lower animals within the limits of reliable medical judgment and do not cause undue toxicity, irritation, allergic reactions, etc., in a reasonable benefit/risk ratio. In one embodiment, the pharmaceutically acceptable salt of the valproic acid comprises sodium valproate.

In the present invention, the dextromethorphan can also be used to improve the memory of the subject. Therefore, the valproic acid taken together with the dextromethorphan also has the effect of improving the memory of the subject. In one embodiment, the active ingredient of the composition for improving the memory of the subject further comprises dextromethorphan.

In another embodiment, the memory comprises verbal memory, spatial memory, immediate memory, delayed memory, and working memory.

As used herein, the term "improve" or "improving" means that in addition to increasing cognitive function (sustained attention and memory), it also includes slowing, stopping, or reversing the progression of cognitive deficits.

In one embodiment, the subject is a normal subject or a normal human.

In another specific embodiment, the subject suffers from a psychiatric disorder, or a disease related to neuronal apoptosis or neurodegeneration. In one preferred embodiment, the psychiatric disorder comprises bipolar disorder, schizophrenia, attention deficit hyperactivity disorder (ADHD), or depression. In a preferred embodiment, the disease related to neuronal apoptosis or neurodegeneration comprises stroke, alzheimer's disease, huntington's disease, parkinson's disease, pick's disease, creutzfeldt-jakob's disease, amyotrophic lateral sclerosis-parkinsonism-dementia complex, wilson's disease, multiple sclerosis, progressive supranuclear palsy, corticobasal degeneration, dementia, amyotrophic lateral sclerosis, epilepsy, transient ischemic attack, myocardial ischemia, head injury, spinal cord injury, or hypoxia. In another embodiment, the subject suffers from bipolar disorder. In a preferred embodiment, the bipolar disorder is bipolar II disorder. In some aspects, childhood-onset fluency disorder, psychiatric disorder, and diseases related to neuronal apoptosis or neurodegeneration cause impairment in the cognitive function of the subject. Therefore, the sustained attention deficit and memory disorder (e.g. memory degradation or decline) of the subject are caused by childhood-onset fluency disorder, psychiatric disorder, mood disorders, neurological conditions, neuronal apoptosis or neurodegeneration.

As used herein, the term "subject" refers to animals. In a preferred embodiment, the subject is a mammal. In a more preferred embodiment, the subject is a human.

In the present invention, an effective amount of the dextromethorphan, valproic acid or sodium valproate is administered to the subject for treating. As used herein, the term "effective amount" refers to the amount of a pharmaceutical ingredient which substantially induces, promotes or results in a desired therapeutic effect. In the present invention, the effective amount of the dextromethorphan is less than 30 mg/day. In one embodiment, the effective amount of the dextromethorphan ranges from 0.1 to 30 mg/day. In a preferred embodiment, the effective amount of the dextromethorphan ranges from 0.5 to 25 mg/day. In a more preferred embodiment, the effective amount of the dextromethorphan ranges from 1 to 20 mg/day. Therefore, the effective amount of the dextromethorphan administered to the subject allows the level of dextromethorphan in the plasma of the subject to be between 10-50 ng/ml (0.05-0.2 µM).

In another embodiment, the effective amount of the valproic acid or sodium valproate ranges from 0.1 to 2500 mg/day. In a preferred embodiment, the effective amount of the valproic acid or sodium valproate ranges from 100 to 2000 mg/day. In a more preferred embodiment, the effective amount of the valproic acid or sodium valproate ranges from 500 to 1000 mg/day. In another embodiment, the effective amount of the valproic acid or sodium valproate ranges from 500 to 2500 mg/day. Therefore, the effective amount of the valproic acid or sodium valproate administered to the subject allows the level of dextromethorphan in the plasma of the subject to be between 50-100 µg/dl.

The drug of the present invention further comprises a pharmaceutically acceptable carrier. As used herein, the term "pharmaceutically acceptable carrier" refers to a diluent or vehicle which is used to enhance the delivery and/or pharmacokinetic properties of a pharmaceutical ingredient with which it is formulated, but has no therapeutic effect of its own, nor does it induce or cause any undesirable or untoward effect or adverse reaction in the subject. Therefore, the active ingredient (dextromethorphan, valproic acid or sodium valproate) of the present invention can be formulated readily by using pharmaceutically acceptable carriers well known in the art into dosages suitable for oral administration, which is also preferred for the practice of the present invention. Such carriers enable the compounds of the present invention to be formulated in dosage forms such as tablets, lozenges, pills, capsules, liquids, gels, syrups, slurries, suspensions and the like, for oral ingestion by a patient to be treated. These carriers may be selected from sugars, starches, cellulose and its derivatives, malt, gelatin, talc, calcium sulfate, vegetable oils, synthetic oils, polyols, alginic acid, phosphate buffered solutions, emulsifiers, isotonic saline, and pyrogen-free water.

In some aspects, the unit dose of dextromethorphan in each tablet or lozenges is 0.1, 0.5, 1, 5, 10, 15 or 30 mg when the composition is formulated in the dosage form of tablets or lozenges. The above described unit dose of dextromethorphan is designed to approach or meet the daily requirement of less than 30 mg/day of dextromethorphan when one or two tablets/lozenges are administered to the subject each day. In addition, the unit dose of sodium valproate in each tablet or lozenge is 0.1, 10, 50, 100, 250, 500 or 1000 mg. The above described unit dose of sodium valproate is designed to approach or meet the daily requirement of 0.1-2500 mg/day of sodium valproate when one or two tablets/lozenges are administered to the subject each day.

In various embodiments, the drug is administered in a form suitable for use by parenteral, transdermal, oral, and topical routes. In a preferred embodiment, the drug is administered by oral route.

In one embodiment, the drug is administered daily for at least one week. In a preferred embodiment, the drug is administered daily for at least two weeks. In a more preferred embodiment, the drug is administered daily for at least four weeks. In another embodiment, the drug is administered daily for at least six weeks. In a preferred embodiment, the drug is administered daily for at least eight weeks. In one embodiment, the drug is administered continuously for at least twelve weeks.

### EXAMPLES

The present invention recruited patients diagnosed with bipolar II disorder (BD-II). The patients were diagnosed by psychiatrists using the improved Chinese version of the Schedule of Affective Disorder and Schizophrenia-Life Time (SADS-L) with good inter-rater reliability and the Diagnostic and Statistical Manual of Mental Disorders, Fourth Edition, Text Revision (DSM-IV-TR) to confirm that the patient meets the integration points of bipolar II disorder in the DSM-IV-TR.

The purpose of the test of the present invention was to test whether dextromethorphan (DM) and sodium valproate (trade name: Depakine) could improve the cognitive function of patients. Therefore, the patients with bipolar II disorder in the present invention were divided into two groups: one group was the patients taking dextromethorphan and sodium valproate together (BD_{DM}), and the other group was the patients taking sodium valproate and placebo (BD_{Pl}). Before the patients of the two group took the drug, the present invention first used the following evaluation test to perform a basic evaluation of the patients of the two groups.

### (A) Efficacy evaluation test:

### (1) Hamilton Depression Rating Scale (HDRS)

For patients with bipolar disorder in the depression phase, the severity of depression was assessed by the HDRS at the time point for taking the drug before performing the trial (baseline) and each follow-up visit during the trial. The present invention adopted a version of HDRS with 17-items which comprised depressed mood, guilt, suicidal thought or action, insomnia initial, insomnia middle, insomnia late, work and interests (assessing pleasure and functioning), motor retardation, motor agitation, psychic anxiety, somatic anxiety, appetite, tiredness, sexual interest, hypochondriasis, weight loss, and insight.

### (2) Young Mania Rating Scale (YMRS)

For the patients with bipolar disorder in the hypomanic phase or the mania phase, the severity of mania would be assessed by YMRS at the time point for taking the drug before performing the trial (baseline) and each follow-up visit during the trial. YMRS could be applied to the assessment of the mania, and YMRS had 11 items: elevated mood, increased motor activity/energy, sexual interest, sleep, irritability, speech, language/thought disorder, content, disruptive/aggressive behavior, appearance, and insight.

### (B) Neuropsychological test:

### (1) Wechsler Memory Scale-III (WMS-III) (Chinese version)

The present invention used WMS-III (Chinese version) to measure the main index scores such as verbal memory, spatial memory, immediate memory, delayed memory, and working memory through various levels of memory, and the main index scores were used to present multiple degrees of memory of the patients. The split-half reliability coefficients of the main index of WMS-III (Chinese version) was between 0.74 and 0.96, and the median was 0.90. The mean of test-retest interval was 36.57 days, the retest reliability coefficients for the main index scored ranged from 0.47 to 0.83, and the median was 0.71.

### (2) Continuous Performance Test (CPT)

The present invention used the computer version of Conner's CPT II 20 which was used to detect the sustained attention of the patients. In the reliability of Conner's CPT II, the split-half reliability ranged from 0.66 to 0.95, and the retest reliability after three months was 055-0.84. For Han Chinese living in Taiwan, CPT has good reliability and validity.

According to the above evaluation and tests, the table 1 showed the basic information of the patients at the baseline.

**Table 1. Comparison of demographic information of each group at the baseline**

| | BD_{DM} (cases: 113) | BD_{Pl} (cases: 77) | Statistical value (X²/F,Z(p)) |
|---|---|---|---|
| age | 27.35 (8.03) | 29.41 (11.10) | 3.00 (.09) |
| Gender (male ratio) | 55.9% | 54.5% | 0.05 (.83) |
| HDRS | 14.37 (8.12) | 13.18 (8.04) | 1.01 (.31) |
| YMRS | 10.19 (5.52) | 11.34 (5.66) | 2.06 (.15) |

| CPT | | | |
|---|---|---|---|
| Detection rate | 53.80 (8.78) | 55.74 (6.74) | 1.39 (.24) |
| Omission rate | 64.16 (40.92) | 69.13 (62.32) | 0.13 (.72) |
| Impulsive errors | 55.84 (10.95) | 56.73 (10.11) | 0.20 (.59) |
| Hit rate | 50.02 (13.10) | 48.75 (14.78) | 0.44 (.51) |
| Variability | 53.62 (15.34) | 54.89 (18.47) | |

| WMS | | | |
|---|---|---|---|
| Verbal memory | 94.56 (21.54) | 89.93 (17.35) | 3.96 (.05) |
| Visual memory | 99.98 (24.53) | 98.14 (21.16) | 1.84 (.17) |
| General memory index | 96.22 (21.39) | 87.38 (24.58) | 4.96 (.03) |
| Focus | 100.37 (20.97) | 99.97 (18.76) | 2.51 (.12) |
| Delayed memory | 98.33 (21.10) | 96.87 (17.93) | 2.03 (.16) |

Both groups were treated with drug. The course of treatment for the group taking dextromethorphan and sodium valproate (BD_{DM} group) was 1 to 30 mg of dextromethorphan and 500 to 2500 mg of sodium valproate per day for 12 weeks. The course of treatment for the group taking sodium valproate and placebo (BD_{Pl} group) was 500-2500 mg of sodium valproate and placebo per day for 12 weeks. After the end of the treatment, the concentrations of dextromethorphan in the plasma of the patients were about 10-50 ng/ml (0.05-0.2 µM), and the concentrations of sodium valproate in the plasma were about 50-100 µg/dl.

During the treatment, the patients in the two groups were visited and tested with HDRS and YMRS every two weeks. The tests of CPT and WMS were performed only in the baseline and the end of the 12-week treatment. Therefore, the efficacy tests would last for 12 weeks.

During the 12-week follow-up of the efficacy tests, the data from some patients were excluded by the present invention because of some factors and conditions. Therefore, the statistics of the efficacy evaluation results of the two groups after the end of the 12-week treatment were shown in Table 2.

**Table 2. Comparison of demographic information of each group in the baseline and the end of the 12-week treatment**

| | BD_{DM} (Cases: 103) | | Statistic/p-value (Z(p)^{a}) | BD_{Pl} (Cases: 57) | | Statistic/p-value (T(p)^{b}) |
|---|---|---|---|---|---|---|
| | Baseline | After treatment (12 weeks) | | Baseline | After treatment (12 weeks) | |
| age | 30.54 | | - | 31.70 | | - |
| gender (Male ratio) | 55% | | | 54.5% | | |
| HDRS | 13.56 (8.04) | 7.65 (4.98) | 7.03 (<.0001) | 13.88 (8.56) | 8.84 (5.42) | 4.17 (<.0001) |
| YMRS | 10.90 (5.79) | 5.16 (3.13) | 9.75 (<.0001) | 10.75 (5.64) | 5.40 (3.98) | 6.41 (<.0001) |

| CPT | | | | | | |
|---|---|---|---|---|---|---|
| Omission rate | 73.56 (58.20) | 60.64 (38.65) | 3.09 (<.0001) | 60.82 (48.59) | 62.22 (41.41) | -0.17 (.86) |
| Impulsive errors | 57.24 (12.10) | 53.89 (13.01) | 2.96 (<.0001) | 57.59 (12.21) | 56.41 (12.97) | 0.71 (.48) |
| Hit rate | 48.92 (14.22) | 46.74 (12.52) | 1.95 (.05) | 43.89 (15.41) | 44.90 (12.88) | -0.62 (.54) |
| Variability | 55.57 (16.57) | 52.06 (15.40) | 2.31 (.02) | 52.31 (16.45) | 51.28 (14.35) | 0.51 (.61) |
| Detection rate | 54.40 (9.12) | 51.28 (11.80) | 3.25 (.002) | 55.50 (6.94) | 53.22 (10.20) | 1.63 (.11) |

| WMS | | | | | | |
|---|---|---|---|---|---|---|
| Verbal | 83.98 | 90.77 | 3.77 | 77.96 | 86.02 | 4.51 |
| memory | (26.83) | (27.14) | (<.0001) | (25.12) | (26.99) | (<.0001) |
| Visual memory | 90.16 (29.16) | 99.18 (30.51) | 3.93 (<.0001) | 82.82 (29.09) | 93.86 (29.70) | 3.73 (<.0001) |
| General memory index | 94.93 (22.89) | 104.12 (24.87) | 4.25 (<.0001) | 91.00 (25.52) | 103.42 (24.08) | 6.05 (<.0001) |
| Focus | 93.49 (23.36) | 95.94 (21.38) | 0.27 (.03) | 88.84 (23.36) | 92.95 (25.19) | 0.97 (.01) |
| Delayed memory | 89.68 (24.39) | 100.10 (24.16) | 6.65 (<.0001) | 86.95 (22.42) | 95.11 (25.00) | 4.39 (<.0001) |

As shown in Table 2, there was no correlation between the changes in HDRS and YMRS scores and the changes in CPT and WMS scores. The results indicated that dextromethorphan and sodium valproate could also be used to improve the cognitive functions of normal humans.

According to the scores of CPT in Table 2, there was no improvement in sustained attention in the BD_{Pl} group (taking sodium valproate and placebo) after the end of the course of treatment. However, the BD_{DM} group (taking dextromethorphan and sodium valproate) showed a significant improvement in sustained attention after the end of the course of treatment. Because the difference in the effect between the two groups was dextromethorphan treatment, the results indicated that the improvement in sustained attention was due to the efficacy of dextromethorphan.

According to the scores of WMS in Table 2, the BD_{Pl} group (taking sodium valproate and placebo) showed significant improvement in memory after the end of the course of treatment. The BD_{DM} group (taking dextromethorphan and sodium valproate) also showed significant improvement in memory after the end of the course of treatment. This result indicated that sodium valproate, dextromethorphan, or a combination thereof had the effect of improving memory.

## Claims

1. A composition for use in a method of improving memory of a subject, wherein the active ingredient of the composition comprises valproic acid or a pharmaceutically acceptable salt thereof.

2. The composition for use according to claim 1, wherein the pharmaceutically acceptable salt of the valproic acid comprises sodium valproate.

3. The composition for use according to claim 1, wherein the composition further comprises dextromethorphan.

4. The composition for use according to claim 1, wherein the memory comprises verbal memory, spatial memory, immediate memory, delayed memory, and working memory.

5. The composition for use according to claim 1, wherein the subject suffers from a psychiatric disorder, or a disease related to neuronal apoptosis or neurodegeneration.

6. The composition for use according to claim 5, wherein the psychiatric disorder comprises bipolar disorder, schizophrenia, attention deficit hyperactivity disorder, or depression.

7. The composition for use according to claim 5, wherein the disease related to neuronal apoptosis or neurodegeneration comprises stroke, alzheimer's disease, huntington's disease, parkinson's disease, pick's disease, creutzfeldt-jakob's disease, amyotrophic lateral sclerosis-parkinsonism-dementia complex, wilson's disease, multiple sclerosis, progressive supranuclear palsy, corticobasal degeneration, dementia, amyotrophic lateral sclerosis, epilepsy, transient ischemic attack, myocardial ischemia, head injury, spinal cord injury, or hypoxia.

8. The composition for use according to claim 1, wherein the effective amount of the valproic acid ranges from 500 to 2500 mg/day.
